# FASCICULE DE BREVET EUROPEEN

(11) **EP 1 764 368 B1**
(45) Date de publication et mention de la délivrance du brevet: **14.07.2010**
(21) Numéro de dépôt: 06124480.2
(22) Date de dépôt: 10.04.1998
(51) Int. Cl.: C07D 519/04, A61K 31/475, A61P 35/00

(54) **Dérivé halogéné antimitotique d'alcaloïde de Vinca**
Antimitotisches halogeniertes Derivat eines Vinca- Alkaloids
Antimitotic halogenated derivative of Vinca alkaloid

(30) Priorité: 10.04.1997 FR 9704398
(43) Date de publication de la demande: 21.03.2007
(62) Demande divisionnaire de: 03017052.6
(73) Titulaire: PIERRE FABRE MEDICAMENT PRODUCTION, 92100 Boulogne Billancourt (FR)
(72) Inventeur: Duflos, Alain, DÉCÉDÉ (FR); Fahy, Jacques, 81290, LABRUGUIERE (FR); Thillaye Du Boullay, Valérie, 81600 Montans (FR); Barret, Jean-Marc, 81100, Castres (FR); Hill, Bridget, NW 19BX London (GB)
(74) Mandataire: Le Forestier, Eric

(56) Documents cités:
- FR-A- 2 707 988
- DATABASE EMBASE [Online] ELSEVIER SCIENCE PUBLISHERS, AMSTERDAM, NL; 1995, SMITH G A: "Current status of vinorelbine for breast cancer" XP002418158 Database accession no. EMB-1995249975 & ONCOLOGY 1995 UNITED STATES, vol. 9, no. 8, 1995, pages 767-773, ISSN: 0890-9091
- JACQUES FAHY ET AL: "Vinca alkaloids in superacidic media: a method for creating a new family of antitumor derivatives." JOURNAL OF THE AMERICAN CHEMICAL SOCIETY., vol. 119, no. 36, 10 septembre 1997 (1997-09-10), pages 8576-8577, XP002072890 DC US -& "Supporting Information"[Online] 10 septembre 1997 (1997-09-10), XP002418156 Extrait de l'Internet: URL:http://pubs.acs.org/subscribe/journals /jacsat/suppinfo/119/i36/ja971864w/ja8576. pdf> [extrait le 2007-02-02]

## Description

Les alcaloïdes dimères du *Catharanthus roseus* et leurs dérivés, couramment appelés alcaloïdes de Vinca, sont largement utilisés en chimiothérapie anticancéreuse depuis une trentaine d'années. Ils sont représentés par quatre produits :
- deux composés naturels, la vinblastine et la vincristine,
- deux produits d'hémisynthèse, la vindésine obtenue à partir de la vinblastine, et la vinorelbine, synthétisée à partir des alcaloïdes monomères précurseurs, catharanthine et vindoline.

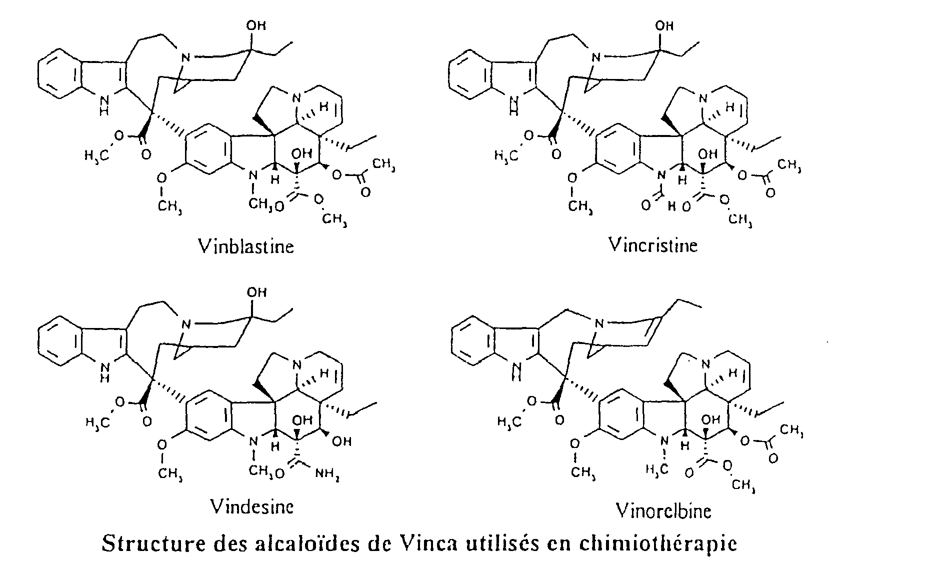

Dans le cadre de nos travaux de recherche visant à obtenir de nouveaux dérivés de cette famille pouvant conduire à de nouvelles applications en chimiothérapie anticancéreuse, nous avons adopté une démarche originale consistant à utiliser la réactivité exceptionnelle des milieux superacides, susceptible d'induire de profondes modifications dans ces molécules hautement fonctionnalisées.

Nous avons ainsi déjà préparé une série de composés difluorés en position 20', qui sont inaccessibles aujourd'hui par les méthodes classiques de synthèse (brevet FR 2 707 988 du 21.07.93, WO 95/03312), et dont les propriétés pharmacologiques sont particulièrement intéressantes.

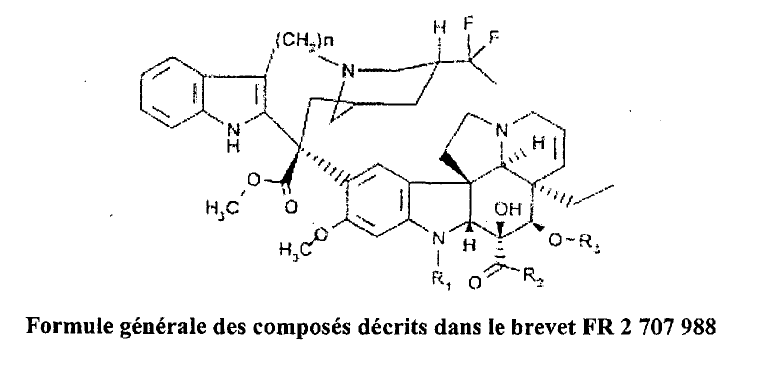

L'application de cette chimie inhabituelle à ce type de molécules complexes nous a permis de préparer le ditartrate de vinflunine, également inaccessible par la chimie classique.

La présente invention, réalisée au Centre de Recherche Pierre Fabre, a pour objet le ditartrate de vinflunine et son application en thérapeutique.

L'invention concerne un sel de la vinflunine avec un acide pharmaceutiquement acceptable, l'acide tartrique.

La 20',20'-difluoro-3',4'-dihydrovinorelbine ou vinflunine est un composé revendiqué dans le brevet FR 2 707 988 du 21.07.93 (WO 95/03312).

Le ditartrate de vinflunine peut être préparé comme indiqué ci-après :

Le dérivé de formule ci-dessous est dissous dans le dérivé chloré, de préférence le chloroforme ou le 2,2-dichloropropane, ou un mélange contenant du chloroforme ou du 2,2-dichloropropane, et ajouté au milieu superacide, en maintenant la température de ce dernier entre -45 et -35°C. Le dérivé difluoré, 4'-déoxy-20',20'-difluorovinblastine, constitue alors le produit final majoritaire de la réaction. Le dosage par CLHP analytique indique une proportion de ce composé de l'ordre de 50%.

Cette réaction est illustrée dans le schéma suivant :

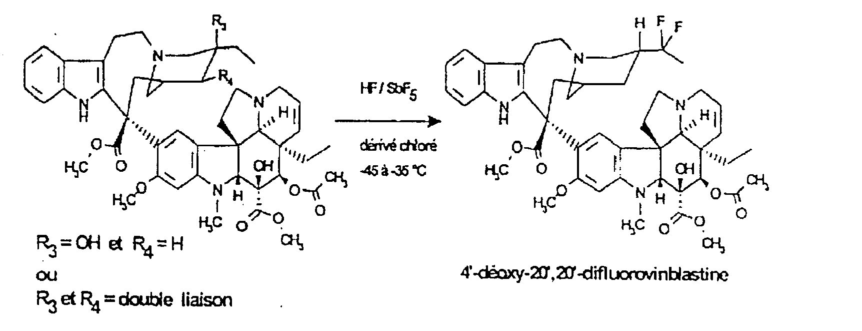

D'autres agents générateurs d'ions superélectrophiles comme le tétrabromure de carbone CBr₄, le dibromométhane CH₂Br₂ ou le tribromure de bore BBr₃ conduisent également à la 4'-déoxy-20',20'-difluorovinblastine de façon majoritaire.

Cette méthode constitue donc un procédé de préparation de la 4'-déoxy-20',20'-difluorovinblastine, avec un rendement supérieur à celui obtenu par la technique antérieure.

La 4'-déoxy-20',20'-difluorovinblastine obtenue suivant cette voie peut ensuite subir une contraction de son cycle C', comme décrit ci-dessus, par réaction avec la N-bromosuccinimide dans le chlorure de méthylène en présence d'un acide comme l'acide trifluoroacétique à température inférieure à -10 °C. Après neutralisation en milieu basique, le composé intermédiaire, 9'-bromoindolénine de la 4'-déoxy-20',20'-difluorovinblastine, peu stable et non isolé, subit une hydrolyse de préférence dans un mélange [chlorure de méthylène : eau : tétrahydrofuranne], en présence ou non de tétrafluoroborate. De la même façon que ci-dessus, l'addition d'AgBF₄ permet d'accroître la vitesse de la réaction.

Le composé majoritaire obtenu avec un rendement de l'ordre de 80% correspond à la 20',20'-difluoro-3',4'-dihydrovinorelbine ou vinflunine. Le schéma ci-dessous résume cette réaction :

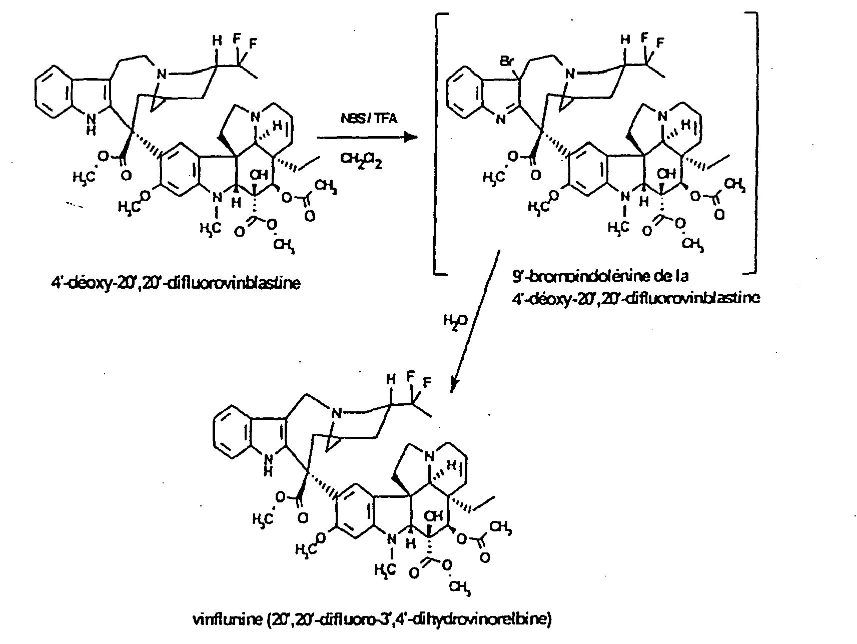

L'enchaînement de ces deux étapes constituent donc un procédé de synthèse de la vinflunine, plus avantageux que le précédent en ce sens que :
1) la réaction de fluoration en milieu superacide est effectuée à partir de composés naturels comme la vinblastine ou la 3',4'-anhydrovinblastine, accessibles directement par extraction des feuilles de *Catharanthus roseus,* et non plus à partir de la vinorelbine, produit d'hémisynthèse plus onéreux,
2) le rendement de la réaction de difluoration par ce nouveau procédé est de l'ordre de 50% contre 25% avec le procédé précédent,
3) la purification par chromatographie des composés obtenus, notamment de la 4'-déoxy-20',20'-difluorovinblastine est moins délicate que dans le cas précédent.

L'exemple suivant illustre l'invention, sans toutefois en limiter la portée. Les caractéristiques spectroscopiques confirment la structure du composé obtenu selon l'invention.

### Exemple 1:

### 20',20'-difluoro-3',4'-dihydrovinorelbine (vinflunine).

On dissout 90 g (0,108 mole) de 4'-déoxy-20',20'-difluorovinblastine dans 800 ml de chlorure de méthylène qu'on refroidit à -45°C, et auxquels on ajoute 10,4 ml (0,135 mole) d'acide trifluoroacétique. On place le montage à l'abri de la lumière, et on y verse durant environ 30 minutes une solution composée de 19,3 g (0,108 mole) de N-bromosuccinimide dans un mélange de 200 ml de chlorure de méthylène et 10,4 ml (0,135 mole) d'acide trifluoroacétique, en veillant à ce que la température du milieu ne remonte pas au-dessus de -45°C.

Après 30 minutes, on neutralise le mélange par addition de 300 ml d'une solution à 10% de NaHCO₃, puis aussitôt une solution de 23,4 g (0, 12 mole) de tétrafluoroborate d'argent dans un mélange de 300 ml de tétrahydrofurane et 100 ml d'eau. On laisse revenir à température ambiante sous agitation pendant environ deux heures.

Après filtration, la phase organique est séparée, lavée par deux fois 150 ml d'eau. Après séchage sur MgSO₄, la solution est évaporée et le résidu est dissous dans 500 ml de méthanol puis évaporé pour obtenir 97 g de résidu sec, contenant 88% de 20',20'-difluoro-3',4'-dihydrovinorelbine (vinflunine).

La purification est effectuée par CLHP préparative sur silice greffée C₁₈ (granulométrie 15-25 µ), avec un éluant composé d'eau, d'acide acétique, d'acétate d'ammonium, de méthanol et d'acétonitrile. Les fractions purifiées sont concentrés à moitié, amenées à pH = 7 et extraites deux fois par du toluène. La phase organique est lavée trois fois par de l'eau distillée, puis dosée par HPLC analytique.

La vinflunine, en solution dans le toluène, est extraite par une solution aqueuse contenant exactement deux équivalents (mole) d'acide tartrique. La phase aqueuse est ensuite lyophilisée pour obtenir le ditartrate de vinflunine.

Comme les alcaloïdes antitumoraux du *Catharanthus roseus,* le composé préparé suivant l'invention est un « poison du fuseau mitotique ».

Cette propriété a été confirmée par la mesure de l'inhibition de la polymérisation de la tubuline en microtubules en présence du composé de l'invention, en suivant la méthode décrite par R. C. Weisenberg (Science 177, 1196-7, 1972).

Compte-tenu de cette propriété pharmacologique caractéristique des alcaloïdes de Vinca, le composé de la présente invention peut être utilisé en chimiothérapie anticancéreuse.

Les préparations pharmaceutiques contenant ce principe actif peuvent être mises en forme pour l'administration par voie orale, intraveineuse ou sous-cutanée, de manière classique, bien connue de l'homme du métier.

## Revendications

1. Ditartrate de vinflunine.

2. Préparation pharmaceutique contenant le ditartrate de vinflunine selon la revendication 1.

3. Préparation pharmaceutique selon la revendication 2, **caractérisée en ce que** qu'elle se trouve sous une forme pour l'administration par voie orale, intraveineuse, ou sous-cutanée.

4. Ditartrate de vinflunine selon la revendication 1 pour son utilisation en tant que médicament.

5. Ditartrate de vinflunine selon la revendication 1 pour son utilisation en tant que médicament destiné à la chimiothérapie anticancéreuse.

6. Utilisation du ditartrate de vinflunine selon la revendication 1 pour la préparation d'un médicament destiné à la chimiothérapie anticancéreuse.

## Claims

1. Vinflunine ditartrate.

2. Pharmaceutical preparation containing the vinflunine ditartrate according to Claim 1.

3. Pharmaceutical preparation according to Claim 2, **characterized in that** it is in a form for oral, intravenous or subcutaneous administration.

4. Vinflunine ditartrate according to Claim 1, for use thereof as a medicinal product.

5. Vinflunine ditartrate according to Claim 1, for use thereof as a medicinal product intended for anticancer chemotherapy.

6. Use of the vinflunine ditartrate according to Claim 1, for preparing a medicinal product intended for anticancer chemotherapy.

## Patentansprüche

1. Vinflunin-Ditartrat.

2. Pharmazeutisches Präparat, enthaltend Vinflunin-Ditartrat nach Anspruch 1.

3. Pharmazeutisches Präparat nach Anspruch 2, **dadurch gekennzeichnet, dass** es in einer Form für die Verabreichung auf oralem, intravenösem oder subkutanem Weg vorliegt.

4. Vinflunin-Ditartrat nach Anspruch 1 für dessen Verwendung als Medikament.

5. Vinflunin-Ditartrat nach Anspruch 1 für dessen Verwendung als Medikament, das für die Antikrebs-Chemotherapie bestimmt ist.

6. Verwendung von Vinflunin-Ditartrat nach Anspruch 1 für die Herstellung eines Medikaments, das für die Antikrebs-Chemotherapie bestimmt ist.
